# DEMANDE DE BREVET EUROPEEN

(11) **EP 0 846 477 A1**
(43) Date de publication de la demande: **10.06.1998**
(21) Numéro de dépôt: 97402880.5
(22) Date de dépôt: 28.11.1997
(51) Int. Cl.: A61N 5/06

(54) **Utilisation d'un chromophore dans une composition destinée à être appliquée sur la peau avant un traitement laser**

(30) Priorité: 05.12.1996 FR 9614954
(71) Demandeur: CENTRE INTERNATIONAL DE RECHERCHES DERMATOLOGIQUES GALDERMA (C.I.R.D. GALDERMA), F-06560 Valbonne (FR)
(72) Inventeur: Mordon, Serge, 59491 Villeneuve D'Ascq (FR); Sumian, Chryslain, 180, Chemin des Combes, 06600 Antibes (FR); Buffard, Karine, 06250 Mougins (FR); Pitre, Franck, 06600 Antibes (FR); Bouclier, Martine, 06560 Valbonne (FR)
(74) Mandataire: Tezier Herman, Béatrice

(57) **Abrégé**

L'invention concerne un procédé pour transformer à la surface de la peau une énergie lumineuse d'un rayonnement laser en énergie thermique utilisant une composition comprenant au moins un chromophore, caractérisé en ce que (1) on applique sur ladite surface de la peau une composition comprenant, dans un support physiologiquement acceptable, au moins un chromophore, cette composition et son épaisseur appliquée présentant à la longueur d'onde d'émission du laser une absorbance telle que l'énergie lumineuse transmise dans la peau n'engendre aucun dommage tissulaire ou cellulaire irréversible non désiré, (2) on applique sur ladite surface de la peau un rayonnement laser, l'irradiation produite par le laser permettant d'obtenir localement au niveau de la composition appliquée la transformation de l'énergie lumineuse en énergie thermique.

Plus particulièrement, l'énergie thermique produite au niveau de la composition durant un seul tir laser va pouvoir se propager par conduction à l'intérieur de la peau, augmentant localement la température à plus de 100 °C afin d'obtenir une volatilisation tissulaire. Après volatilisation tissulaire, une nouvelle peau se reforme présentant un aspect plus jeune et/ou moins disgracieux.

## Description

L'invention a trait à l'utilisation d'un chromophore dans une composition destinée à être appliquée sur la peau avant un traitement laser. L'effet thermique produit lors du traitement laser étant principalement destiné à effectuer une volatilisation tissulaire.

La nature des interactions entre la lumière émise par un laser et le tissu biologique est complexe et dépend de nombreux facteurs. Actuellement, chaque pathologie, désordre ou caractère inesthétique de la peau, nécessite un type de laser spécifique dont le choix dépend essentiellement de la cible à atteindre et de l'effet à produire.

En particulier, la volatilisation des premières couches de la peau (qui peut aller jusqu'au derme) à l'aide d'un laser ou relissage cutané n'est réalisée qu'avec des lasers émettant dans le spectre infrarouge et ayant ainsi une longueur d'onde principalement absorbée par l'eau. Cependant, la répartition de l'eau dans la peau dépend du site visé, du type de peau et de l'âge de la personne à traiter. Il est donc évident que le traitement par laser du relief cutané ciblant l'eau intracellulaire n'est pas reproductible d'une personne à une autre. On peut citer comme exemples de lasers émettant dans le spectre infrarouge et ciblant l'eau intracellulaire, les lasers : CO₂ (10,6 µm), Er:YAG (2,94 µm) et Ho:YAG (2,12 µm).

Les lasers émettant dans le spectre visible ou dans l'infrarouge proche (longueurs d'onde de 400 nm à 1000 nm), ayant une profondeur de pénétration importante dans la peau, sont principalement utilisés pour le traitement des lésions de type vasculaires ou pigmentaires, mais ne peuvent pas être utilisés pour un relissage cutané. On peut citer comme exemples de lasers émettant dans le spectre visible, le laser à colorant pulsé (585 nm) pour le traitement des lésions vasculaires et le laser Nd:YAG doublé (532 nm) pour le traitement des lésions pigmentaires.

La réponse clinique et histologique de la peau à l'irradiation lumineuse varie considérablement selon le type de laser et la longueur d'onde utilisée. Au niveau de la cible, plusieurs effets peuvent être générés, ils dépendent directement de la nature du chromophore (coefficient d'absorption à une longueur d'onde donnée, structure et composition chimique ...), de l'énergie par unité de surface (ou fluence) et de la puissance par unité de surface (ou irradiance). L'étude de l'interaction du rayonnement avec les tissus biologiques permet de dissocier plusieurs mécanismes intervenants. Dans le domaine de la dermatologie, l'utilisation des lasers est principalment basée sur deux types de mécanisme, soit l'effet thermique où l'énergie lumineuse est transformée en énergie thermique, soit l'effet mécanique où la lumière crée des ondes de choc.

L'effet thermique résulte de l'absorption par le tissu biologique de l'énergie lumineuse transportée par le faisceau laser et sa dégradation locale en énergie thermique. Pour une longueur d'onde donnée, l'échauffement des tissus est fonction de la fluence et de l'irradiance. Suivant l'importance de l'échauffement, on peut observer une coagulation, une carbonisation ou une volatilisation des cellules constituant le tissu biologique. L'action thermique du laser peut se résumer en trois actions principales selon le degré et le temps d'échauffement tissulaire :
- L'hyperthermie signifie une élévation modérée de la température, portant le tissu à des températures de 41 à 44 °C pendant plusieurs minutes. Cette action conduit à une altération cellulaire avec disparition des membranes et dénaturation des enzymes.
- La coagulation correspond à une nécrose sans destruction tissulaire immédiate. La température atteinte par le tissu se situe entre 50 et 100 °C, pendant une durée de l'ordre de la seconde. Cette action produit une dessiccation avec rétraction des tissus par dénaturation des protéines et du collagène. La nécrose est irréversible sans perte de substance immédiate.
- La volatilisation correspond à une perte de substance. Les divers constituants tissulaires partent en fumée. La température atteinte se situe entre 100 et 1000 °C, pendant une durée relativement courte (de l'ordre du dixième de seconde). Entre 100 et 300 °C, l'ablation tissulaire est réalisée par vaporisation explosive dû à la rupture des vacuoles.
La transition thermique entre la zone irradiée et la zone saine se faisant graduellement, l'examen histologique permet de différencier trois zones qui correspondent, de la zone irradiée la plus proche à la plus éloignée, à : une zone de carbonisation ou zone de volatilisation tissulaire (libération du carbone intracellulaire à 150°C), une zone de coagulation et une zone d'hyperthermie.

Lors de la dégradation de l'énergie thermique dans le tissu, il peut être important d'ajuster la durée de l'irradiation laser à une durée appelée temps de relaxation thermique afin de limiter le dommage thermique créé aux tissus adjacents. Physiquement, ce temps est considéré comme le temps nécessaire au tissu pour réduire son excès de température de 50 % de la température initiale. Si la durée d'irradiation par laser est inférieure à ce temps de relaxation la chaleur ne pourra pas se diffuser à l'intérieur du tissu et restera confinée dans le volume irradié. De plus, si dans ce même temps l'énergie déposée au niveau de la cible est suffisante pour l'élever à une température très supérieure à 100 °C, la vaporisation locale du milieu sera créée. L'expansion de cette bulle de vapeur à l'intérieur du tissu resté froid génère des ondes thermoélastiques de faible amplitude. Ce procédé de photothermolyse sélective est par exemple utilisé pour le traitement des angiodysplasies cutanées : les erythrocytes absorbent le pulse, explosent, se vaporisent et l'expansion rapide de cette vapeur entraînent la rupture du vaisseau avec une extravasation du sang. Cette technique utilisé en surface permet d'obtenir localement l'ablation du tissu visé.
Par ailleurs, l'étude du spectre d'absorption des différents tissus montre que la profondeur de pénétration optique du rayonnement est fonction de la longueur d'onde. Ainsi, la dégradation énergétique en chaleur se produit dans un volume interactif qui dépend essentiellement de la profondeur de pénétration du faisceau (zone irradiée), des coefficients de diffusion et de conductivité thermique des tissus atteints, de la vascularisation locale et de la capacité de la cible à garder la chaleur emmagasinée ou à la perdre.

L'effet thermique, tel que décrit ci-dessus, est généralement obtenu pour une irradiance inférieure à environ 10⁸ W/cm², ce qui correspond à une durée d'émission supérieure ou égale à environ 10⁻⁵ s.

L'effet mécanique repose sur la possibilité de concentrer une grande quantité d'énergie lumineuse sur une surface suffisamment petite et pendant un temps suffisamment bref pour obtenir le claquage optique du milieu. Ce claquage optique induit la formation d'un plasma, c'est à dire d'un gaz fortement ionisé induit par une irradiance supérieure ou égale à environ 10⁸ W/cm² (ce qui correspond à une durée d'émission inférieure ou égale à 10⁻⁷ s, donc une durée 100 fois plus courte que dans le cas d'un effet thermique). A la formation de ce plasma est associée la génération d'ondes de chocs, le phénomène de cavitation et la formation de jet . A la frontière entre le milieu ionisé (plasma) et le milieu externe apparaît un gradient de pression qui induit la formation d'une onde de choc qui va se propager dans les tissus adjacents. A la suite de ce phénomène (50 - 150 ns après le pulse) apparaît la cavitation, c'est à dire la formation de bulles qui suivent pendant quelques centaines de microsecondes un processus oscillatoire d'expansion et de collapses (effondrement de la bulle sur elle-même). Lors de ces collapses, la pression augmentant considérablement à l'intérieur de la bulle, une nouvelle onde de choc est émise.
Enfin, chaque collapse peut induire la formation de jet si la bulle est générée à proximité d'une paroi solide (par exemple à proximité d'un os). Ce jet peut alors être responsable de la détérioration en surface de la paroi solide (érosion localisée du solide).

C'est ainsi qu'il est décrit dans le brevet US 5.423.803 un procédé pour retirer de la peau humaine une partie du stratum corneum à l'aide de lasers émettant dans le spectre infrarouge (Nd:YAG, 1064 nm; CO₂ , 10.6 µm) et ayant une durée d'émission inférieure ou égale à 50 ns. Avant l'irradiation laser, une composition comprenant des chromophores est appliquée sur la peau à traiter. En utilisant soit des ultra-sons, soit le laser, ces chromophores sont placés dans les espaces intercellulaires du stratum corneum. Cette partie de la peau traitée est ensuite irradiée par un faisceau laser présentant une énergie suffisante pour réaliser l'ionisation (après claquage optique) des chromophores. Tel qu'il est décrit ci-dessus, l'ionisation du chromophore entraîne la formation d'ondes de choc (effet mécanique) responsables de la volatilisation des trois premières couches cellulaires du stratum corneum. Cet effet étant basé sur l'émission d'ondes de choc, il présente l'inconvénient de générer aux tissus adjacents à la partie traitée des lésions irréversibles non désirées. Par ailleurs, l'efficacité de ce traitement est limité dans l'espace et dans la qualité par la pénétration des chromophores dans le stratum corneum.

Un des buts de la présente invention est de pouvoir procéder à un relissage cutané à l'aide d'un laser émettant dans le spectre visible.

Un autre but de la présente invention est de rendre reproductible le relissage cutané à l'aide d'un laser émettant dans le spectre infrarouge.

Un autre but est de proposer un traitement évitant la formation de lésions irréversibles non désirées.

Ces buts et d'autres sont atteints par la présente invention qui concerne un procédé pour transformer à la surface de la peau une énergie lumineuse d'un rayonnement laser en énergie thermique utilisant une composition comprenant au moins un chromophore, caractérisé en ce que (1) on applique sur ladite surface de la peau une composition comprenant, dans un support physiologiquement acceptable, au moins un chromophore, cette composition et son épaisseur appliquée présentant à la longueur d'onde d'émission du laser une absorbance telle que l'énergie lumineuse transmise dans la peau n'engendre aucun dommage tissulaire ou cellulaire irréversible non désiré, (2) on applique sur ladite surface de la peau un rayonnement laser, l'irradiation produite par le laser permettant d'obtenir localement au niveau de la composition appliquée la transformation de l'énergie lumineuse en énergie thermique.

Plus particulièrement, l'énergie thermique produite au niveau de la composition durant un seul tir laser va pouvoir se propager par conduction à l'intérieur de la peau, augmentant localement la température à plus de 100 °C afin d'obtenir une volatilisation tissulaire.

Ainsi, la présente invention a également pour objet l'utilisation d'au moins un chromophore dans la fabrication d'une composition destinée à être appliquée topiquement sur la surface de la peau avant une application sur ladite surface de la peau d'un rayonnement laser, l'irradiation produite par le laser permettant d'obtenir localement au niveau de la composition appliquée la transformation de l'énergie lumineuse en énergie thermique, cette énergie thermique permettant d'obtenir une volatilisation tissulaire de la peau se situant sous ladite surface.

Préférentiellement, cette composition et son épaisseur appliquée présentent à la longueur d'onde d'émission du laser une absorbance telle que l'énergie lumineuse transmise dans la peau n'engendre aucun dommage tissulaire ou cellulaire irréversible non désiré.

Après volatilisation tissulaire, une nouvelle peau se reforme présentant un aspect plus jeune et/ou moins disgracieux, ce qui correspond à un relissage cutané. Plus particulièrement, ce procédé peut permettre d'éliminer un caractère inesthétique de la peau, tel que des rides, des ridules, des verrues, des cicatrices atrophiques et/ou hypertrophiques. De plus, ce traitement peut être utilisé seul ou en complément d'un traitement médicamenteux pour traiter des pathologies cutanées, telles que notamment le rhinophyma, une hyperkératose, des hyperproliférations cutanées, une plaque de psoriasis, un cancer cutané, une kératose actinique et des kéloïdes.

Par ailleurs, la volatilisation tissulaire peut permettre d'augmenter la pénétration d'actifs cosmétiques ou pharmaceutiques, plus particulièrement dermatologiques. On applique alors, après l'irradiation et avant la reformation complète de la nouvelle peau, une composition cosmétique ou pharmaceutique comprenant au moins un actif. A titre d'exemples d'actifs, on peut citer les actifs utilisés comme médicament par la voie cutanée en vue d'une délivrance locale et/ou systémique, tels que notamment l'acide rétinoïque et ses dérivés (rétinoïdes), le peroxyde de benzoyl, les antibiotiques, les corticostéroïdes, les antifongiques, la vitamine D3 ou D2 et leurs dérivés.

La présente invention a aussi pour objet un procédé de traitement cosmétique, notamment pour diminuer les rides et les ridules, caractérisé en ce que (1) on applique sur la surface de la peau une composition comprenant, dans un support physiologiquement acceptable, au moins un chromophore, cette composition et son épaisseur appliquée présentant à la longueur d'onde d'émission du laser une absorbance telle que l'énergie lumineuse transmise dans la peau n'engendre aucun dommage tissulaire ou cellulaire irréversible non désirée, (2) on applique sur ladite surface de la peau un rayonnement laser, l'irradiation produite par le laser permettant d'obtenir localement au niveau de la composition appliquée la transformation de l'énergie lumineuse en énergie thermique, cette énergie thermique permettant d'obtenir une volatilisation tissulaire de la peau se situant sous ladite surface.

L'effet thermique, qui correspond à la transformation de l'énergie lumineuse en énergie thermique, est tel que décrit précédemment. Cet effet thermique est donc généralement obtenu par un laser dont l'irradiance est inférieure à environ 10⁸ W/cm², ce qui correspond à une durée d'émission supérieure ou égale à environ 10⁻⁵ s. De préférence, il est obtenu par un laser dont l'irradiance est inférieure ou égale à environ 10⁷ W/cm².

De préférence, l'irradiance est supérieure ou égale à 0,5 W/cm², avantageusement supérieure ou égale à 10 W/cm² et encore plus avantageusement supérieure ou égale à 100 W/cm².

De préférence, la durée d'émission est inférieure ou égale à 100 s et avantageusement inférieure ou égale à 10 s.

Ainsi, l'énergie lumineuse émise par le laser est transformée en énergie thermique au niveau de la composition appliquée sur la peau grâce à l'absorption des chromophores présents dans cette composition.

Plus particulièrement, l'énergie thermique permet de n'obtenir qu'une volatilisation tissulaire du stratum corneum et de l'épiderme. Dans ces conditions, la coagulation du derme, telle que décrite précédemment, peut ou non avoir lieu et ceci dépend de l'énergie thermique transmise à la peau. Dans certains traitements, comme par exemple l'augmentation de pénétration d'actifs, la coagulation du derme superficiel n'est pas nécessaire.

Les chromophores utilisables dans la présente invention sont avantageusement ceux qui permettent à une composition les contenant de présenter à la longueur d'onde d'émission du laser une absorbance telle que l'énergie lumineuse transmise dans la peau n'engendre aucun dommage tissulaire ou cellulaire irréversible non désiré. Plus particulièrement, on peut envisager d'utiliser des chromophores minéraux, tels que le noir de carbone, le graphite, l'oxyde de fer noir et l'oxyde de fer rouge, des chromophores organiques, tels que la mélanine, le vert d'indocyanine, les colorants ou tout autre entité chimique inerte absorbant de façon suffisante à la longueur d'onde considérée (par exemple à 1064 nm : les dérivés de silicium, les dérivés de cholestérol, les phosphates, les sulfates). De préférence, on utilise des chromophores minéraux.

Ces chromophores peuvent être dispersés dans un support huileux et/ou aqueux (émulsions, gels, onguents, dispersions de polymères, tous systèmes vésiculaires, aérosols, suspensions en milieu liquide filmogène ou non, éventuellement présentés en aérosol) ou solubilisés dans tous types de supports physiologiquement acceptables.

De préférence, les chromophores et/ou la composition les comprenant sont choisis et/ou formulée de telle sorte qu'il n'y ait pas pénétration des chromophores à travers la peau. Ainsi, les chromophores peuvent présenter une granulométrie suffisante pour ne pas pénétrer à travers la peau ou la composition comprend des chromophores sous forme d'agrégats ne permettant pas leur pénétration à travers la peau.

Les dommages tissulaires ou cellulaires irréversibles non désirés correspondent notamment à une atteinte des vaisseaux capillaires se situant dans le derme par coagulation de l'hémoglobine ou une destruction irréversible des mélanocytes, des cellules de langerhans, des kératinocytes ou des fibroblastes, plus particulièrement de leurs précurseurs, notamment par volatilisation des chromophores endogènes contenues dans ces cellules ou leurs précurseurs, tels que l'eau, la mélanine ou des protéines.

Tous les types de lasers, émettant soit dans le spectre visible, soit dans le spectre infrarouge, pourront être utilisés en (2) du moment qu'ils permettent de générer un effet thermique. On peut citer comme exemples de lasers émettant dans le spectre visible, le laser à colorant pulsé (585 nm), le laser Rubis (694 nm) et le laser Nd:YAG doublé (532 nm), et pour les lasers émettant dans le spectre infrarouge, les lasers CO₂ (10,6 µm), Er:YAG (2,94 µm), Ho:YAG (2,12 µm) et Nd:YAG (1,06 µm).

L'absorbance de la composition et de son épaisseur appliquée sur la surface de la peau dépend de la longueur d'onde d'émission du laser, mais aussi des caractéristiques physico-chimiques de la formulation. En effet, l'absorbance varie en fonction du type de chromophore, de sa granulométrie, de la qualité de la dispersion de ce même produit, de la concentration, et de la formulation de la composition; ainsi, à titre d'exemple, dans certaines conditions le noir de carbone formulé à 2.5% n'a pas le même spectre d'absorption qu'une formule à 0.25%.

Aussi, pour une concentration donnée de pigment, dans un support donné, et dans les mêmes conditions d'expérimentation, plus on augmente la qualité de dispersion du pigment (utilisation de tricylindre, d'ultraturax, passage par une pâte pigmentaire), meilleur est l'absorption de la lumière.

Dans le cas d'un laser émettant dans le spectre visible ou dans l'infrarouge proche (longueur d'onde inférieure à 1 µm), la composition comprenant au moins un chromophore étant placé entre le rayonnement du laser et la peau permet à la mélanine, l'hémoglobine et l'oxyhémoglobine, qui sont les principaux chromophores endogènes absorbant à ces longueurs d'onde, d'être protégés de l'irradiation laser. Ainsi, la volatilisation tissulaire est effectuée en protégeant les composés endogénes du derme et ne dépend principalement que de la composition utilisée selon l'invention, de l'épaisseur de cette composition sur la peau et bien entendu des paramètres du laser utilisé.

Dans le cas d'un laser émettant dans le spectre infrarouge (longueur d'onde supérieure à 1 µm), la composition comprenant au moins un chromophore exogène étant placé entre le rayonnement du laser et la peau permet que le résultat obtenu sur la peau ne dépende plus de la répartition de l'eau contenue dans les tissus. En effet la répartition de l'eau dans ces tissus dépend à la fois du site visé, du type de peau et de l'âge du patient à traiter. Ainsi, le résultat obtenu ne dépend principalement que de la composition utilisée selon l'invention, de l'épaisseur de cette composition sur la peau et bien entendu des paramètres du laser utilisé.

Ainsi, pour un traitement donné (perte de matière et profondeur à atteindre), le résultat est atteint en définissant, pour l'utilisateur d'un laser spécifique, l'irradiance et la fluence en fonction de la composition et de son épaisseur à appliquer sur la surface de la peau.

Les figures 1 à 6 permettent de mieux illustrer l'invention, sans toutefois en limiter sa portée. Ces figures correspondent à une représentation shématique de la peau d'un rat nu.
Dans la figure 1 : 1 représente le stratum corneum, 2 le stratum granulosum, 3 le stratum spinosum, 4 le stratum germinativum, 5 la membrance basale, 6 le derme et 7 une glande sébacée dans le derme.
La figure 2 correspond à l'étape (1) du procédé : application de la composition A comprenant les chromophores sur la surface de la peau ainsi shématisée.
La figure 3 correspond à l'étape (2) du procédé : application d'un rayonnement lumineux par laser.
La figure 4 représente le volume interactif B de la composition appliquée sur ladite surface de la peau avec l'énergie lumineuse et transformant celle-ci en énergie thermique.
La figure 5 représente le volume C (partie hachurée) chauffé par conduction thermique.
La figure 6 représente une volatilisation tissulaire de la peau, qui ici va jusqu'à la membrane basale.

Plus particulièrement, l'irradiance et la fluence sont choisies pour que la durée d'irradiation par laser soit inférieure au temps de relaxation thermique de la peau traitée. La chaleur peut alors se diffuser à l'intérieur de la peau, mais reste confinée dans un volume réduit, rendant la volatilisation tissulaire plus sélective.

Avantageusement, avant l'application du rayonnement laser, on peut appliquer une composition, plus particulièrement une composition filmogène (composition séchant après application), n'absorbant pas la lumière à la longueur d'onde utilisée par dessus la composition utilisée selon l'invention. Cette addition a pour but de confiner l'énergie libérée à un volume interactif plus réduit et d'amplifier les ondes thermoélastiques émises lors de la volatilisation des tissus. Ainsi, avec un minimum d'énergie lumineuse, le tissu peut être volatilisé tout en limitant les dommages engendrés aux tissus adjacents.

Selon un mode particulier de réalisation de l'invention, on peut relier le laser à un dispositif permettant de déplacer le laser sur une plus grande surface que celle correspondant à la surface du faisceau du laser appliquée sur la peau, afin de pouvoir traiter régulièrement et de façon reproductible ladite surface. Le dispositif utilisé peut être notamment celui décrit dans le brevet US 5330517.

On va maintenant donner, à titre d'illustration et sans aucun caractère limitatif, plusieurs exemples.

Dans ce qui suit ou ce qui précède, les pourcentages donnés sont en poids sauf mention contraire.

### EXEMPLE 1:

| Emulsion H/E à base d'oxyde de fer rouge (5%). | |
|---|---|
| composants | % |
| Lanol CTO (Seppic) alcool cétylstéarylique | 7 |
| Geleol (Gattefossé) stéarate de glycéryle | 2 |
| alcool cétylique | 1,5 |
| DC 200, 300cp polydiméthylsiloxane | 1,5 |
| Polysynlane (NOF) Iso-paraffine hydrogénée | 15,1 |
| Sicovit rouge 30E172 (BASF) Oxyde de fer rouge | 5 |
| glycérine | 20,1 |
| eau | 47,8 |

### EXEMPLE 2:

| Gel aqueux à base de noir de carbone (2.5%) | |
|---|---|
| composants | % |
| Derussol A (Degussa) dispersion aqueuse de noir de carbone | 16,65 |
| eau | 72,85 |
| Aérosil 200 (Degussa) | 7,5 |
| propylène glycol | 2 |
| poloxamère 182 | 1 |

### EXEMPLE 3:

| Onguent à base de noir de carbone (2.5%) | |
|---|---|
| composants | % |
| FW1 (Degussa) noir de carbone | 2,5 |
| Polysynlane (NOF) | 19,4 |
| Dispersant lipophile approprié | 0,63 |
| vaseline codex | 77,47 |

### EXEMPLE 4:

| Solution filmogène à base de noir de carbone (2,28 %) | |
|---|---|
| composants | % |
| Derussol A (Degussa) | 15,2 |
| Eudragit NE 30 D (Rohm and Haas) | 34,7 |
| Eau | 50,1 |

### EXEMPLE 5

### exemple de traitement : le relissage cutané

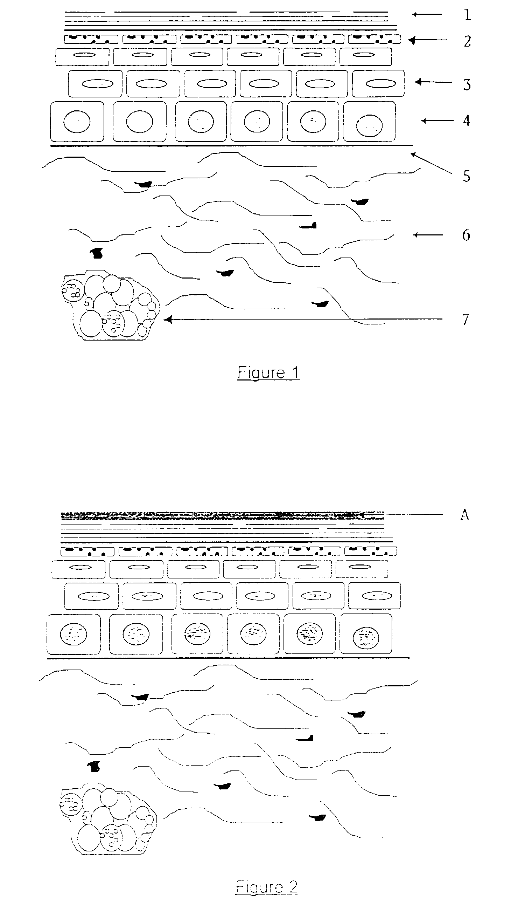

Le relissage cutané par l'addition d'une préparation galénique peut être décrit par les figures 1 à 6.
La Fig. 1 rappelle la structure de l'épiderme et du derme du rat nu.

### Application de la préparation galénique

L'application topique est effectuée au niveau de la surface de la peau (sur le stratum corneum) d'un rat nu. Le chromophore contenu dans la composition reste à la surface du stratum corneum et n'est pas distribué dans celui-ci (Fig. 2).

### Irradiation laser

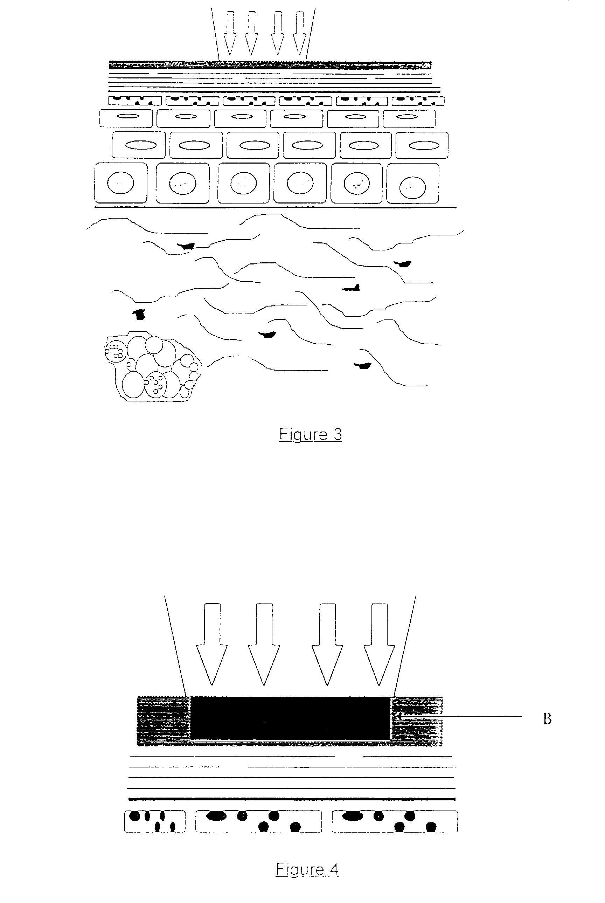

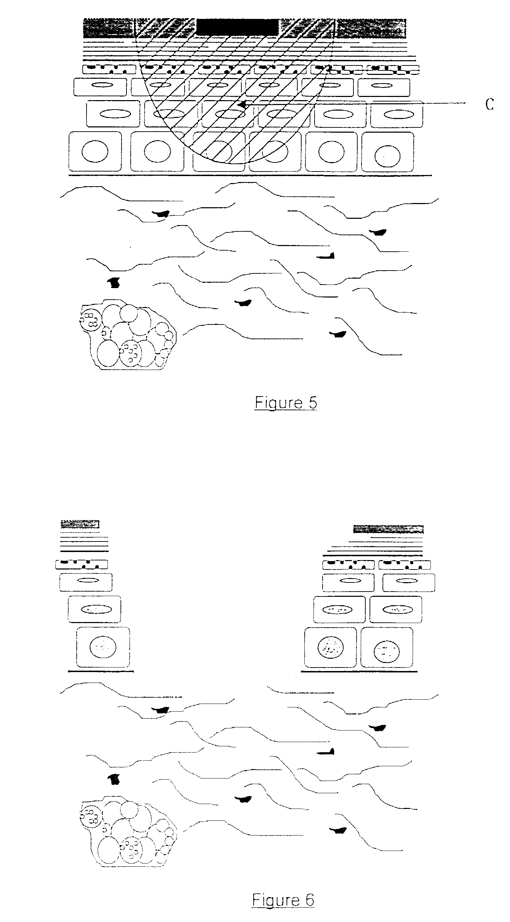

L'étape suivante consiste à irradier la surface de la peau avec un laser Nd:YAG doublé (532 nm) et ayant une durée d'émission supérieure à 1 µs (Fig. 3). Le mécanisme utilisé étant l'effet thermique, l'irradiance est inférieure à 10⁷ W/cm². La composition appliquée (compositions données en exemples 1 ou 2) et son épaisseur appliquée présentent une absorbance telle que l'énergie lumineuse transmise au tissu (épiderme, derme) n'est pas suffisante pour engendrer des dommages tissulaires et cellulaires irréversibles. La composition étant placée entre le rayonnement laser et la peau, la mélanine présente dans l'épiderme ainsi que l'oxyhémoglobine et l'hémoglobine contenues dans les vaisseaux sont protégés de l'irradiation.
En appliquant une épaisseur de composition suffisante (environ 100 µm pour les compositions citées en exemples 1 et 2), l'énergie lumineuse absorbée par le chromophore contenu dans les compositions est transformée localement (au niveau de la composition) en énergie thermique (Fig. 4). La chaleur produite au niveau de la composition durant un seul tir laser va se propager par conduction à l'intérieur de la peau (Fig. 5), augmentant localement la température à plus de 100 °C afin d'obtenir une volatilisation tissulaire (Fig 6).

Plus particulièrement, en appliquant la composition décrite dans l'exemple 2 sur la surface de la peau de rat nu avec une épaisseur d'environ 100 µm, avec une irradiance de 450 W/cm² et une fluence de 25 J/cm², il est possible de coaguler le derme superficiel sur 25 à 50 µm et de réaliser en même temps la volatilisation de l'épiderme jusqu'a la couche basale. Après 8 à 10 jours, la peau du rat se reforme grâce au phénomene de cicatrisation. Par ce phénoméne, le stratum corneum, comme le reste de la peau, se trouve restauré.

L'emploi d'autres chromophores exogénes modifie les conditions d'irradiation de la composition appliquée sur le stratum corneum pour le même resultat que précédemment. A titre d'exemple, l'emploi de la composition décrite dans l'exemple 1 nécessite une irradiance identique (450 W/cm²), mais une fluence quasiment 2 fois plus elevée pour obtenir le même résultat.

## Revendications

1. Procédé pour transformer à la surface de la peau une énergie lumineuse d'un rayonnement laser en énergie thermique utilisant une composition comprenant au moins un chromophore, caractérisé en ce que (1) on applique sur ladite surface de la peau une composition comprenant, dans un support physiologiquement acceptable, au moins un chromophore, cette composition et son épaisseur appliquée présentant à la longueur d'onde d'émission du laser une absorbance telle que l'énergie lumineuse transmise dans la peau n'engendre aucun dommage tissulaire ou cellulaire irréversible non désiré, (2) on applique sur ladite surface de la peau un rayonnement laser, l'irradiation produite par le laser permettant d'obtenir localement au niveau de la composition appliquée la transformation de l'énergie lumineuse en énergie thermique.

2. Utilisation d'au moins un chromophore dans la fabrication d'une composition destinée à être appliquée topiquement sur la surface de la peau avant une application sur ladite surface de la peau d'un rayonnement laser, l'irradiation produite par le laser permettant d'obtenir localement au niveau de la composition appliquée la transformation de l'énergie lumineuse en énergie thermique, cette énergie thermique permettant d'obtenir une volatilisation tissulaire de la peau se situant sous ladite surface.

3. Utilisation selon la revendication précédente, caractérisée en ce que la composition et son épaisseur appliquée présentent à la longueur d'onde d'émission du laser une absorbance telle que l'énergie lumineuse transmise dans la peau n'engendre aucun dommage tissulaire ou cellulaire irréversible non désiré.

4. Utilisation selon l'une des revendications 2 ou 3, caractérisée en ce que la volatilisation tissulaire a pour but d'éliminer un caractère inesthétique de la peau, tel que des rides, des ridules, des verrues, des cicatrices atrophiques et/ou hypertrophiques.

5. Utilisation selon l'une des revendications 2 ou 3, caractérisée en ce que la volatilisation tissulaire a pour but de traiter, éventuellement en complément d'un traitement médicamenteux, des pathologies cutanées, telles que le rhinophyma, une hyperkératose, des hyperproliférations cutanées, une plaque de psoriasis, un cancer cutané, une kératose actinique, des kéloïdes.

6. Utilisation selon l'une des revendications 2 ou 3, caractérisée en ce que la volatilisation tissulaire a pour but d'augmenter la pénétration d'actifs cosmétiques ou pharmaceutiques, plus particulièrement dermatologiques.

7. Utilisation selon l'une des revendications 2 à 6, caractérisée en ce que l'énergie thermique permet de n'obtenir qu'une volatilisation tissulaire du stratum corneum et de l'épiderme.

8. Utilisation selon l'une quelconque des revendications précédentes 2 à 7, caractérisée en ce que l'effet thermique est obtenu par un laser dont l'irradiance est inférieure à environ 10⁸ W/cm², de préférence, il est obtenu par un laser dont l'irradiance est inférieure ou égale à environ 10⁷ W/cm².

9. Utilisation selon l'une quelconque des revendications précédentes 2 à 8, caractérisée en ce que l'effet thermique est obtenu par un laser dont l'irradiance est supérieure ou égale à 0,5 W/cm², avantageusement supérieure ou égale à 10 W/cm² et encore plus avantageusement supérieure ou égale à 100 W/cm².

10. Utilisation selon l'une quelconque des revendications précédentes 2 à 9, caractérisée en ce que les chromophores sont choisis parmi des chromophores minéraux, tels que le noir de carbone, le graphite, l'oxyde de fer noir et l'oxyde de fer rouge, et des chromophores organiques, tels que la mélanine, le vert d'indocyanine, les colorants ou tout autre entité chimique inerte absorbant de façon suffisante à la longueur d'onde considérée.

11. Utilisation selon la revendication précédente, caractérisée en ce que les chromophores sont des chromophores minéraux.

12. Utilisation selon l'une quelconque des revendications précédentes 2 à 11, caractérisée en ce que les chromophores et/ou la composition les comprenant sont choisis et/ou formulée de telle sorte qu'il n'y ait pas pénétration des chromophores à travers la peau.

13. Utilisation selon l'une quelconque des revendications précédentes 2 à 12, caractérisée en ce que, avant application du rayonnement laser, on applique une composition, plus particulièrement une composition filmogène, n'absorbant pas la lumière à la longueur d'onde utilisée par dessus la composition comprenant au moins un chromophore.

14. Utilisation selon l'une quelconque des revendications précédentes 2 à 13, caractérisée en ce qu'on utilise des lasers émettant dans le spectre visible, tels que, plus particulièrement, le laser à colorant pulsé (585 nm), le laser Rubis (694 nm) et le laser Nd:YAG doublé (532 nm).

15. Utilisation selon l'une quelconque des revendications précédentes 2 à 13, caractérisée en ce qu'on utilise des lasers émettant dans le spectre infrarouge, tels que, plus particulièrement, les lasers CO₂ (10,6 µm), Er:YAG (2,94 µm), Ho:YAG (2,12 µm) et Nd:YAG (1,06 µm).

16. Procédé de traitement cosmétique, notamment pour diminuer les rides et les ridules, caractérisé en ce que (1) on applique sur la surface de la peau une composition comprenant, dans un support physiologiquement acceptable, au moins un chromophore, cette composition et son épaisseur appliquée présentant à la longueur d'onde d'émission du laser une absorbance telle que l'énergie lumineuse transmise dans la peau n'engendre aucun dommage tissulaire ou cellulaire irréversible non désirée, (2) on applique sur ladite surface de la peau un rayonnement laser, l'irradiation produite par le laser permettant d'obtenir localement au niveau de la composition appliquée la transformation de l'énergie lumineuse en énergie thermique, cette énergie thermique permettant d'obtenir une volatilisation tissulaire de la peau se situant sous ladite surface.

17. Procédé selon la revendication précédente, caractérisé en ce que l'énergie thermique permet de n'obtenir qu'une volatilisation tissulaire du stratum corneum et de l'épiderme.

18. Procédé selon l'une des revendications 1, 16 et 17, caractérisé en ce que l'effet thermique est obtenu par un laser dont l'irradiance est inférieure à environ 10⁸ W/cm², de préférence, il est obtenu par un laser dont l'irradiance est inférieure ou égale à environ 10⁷ W/cm².

19. Procédé selon l'une des revendications 1, 16 à 18, caractérisé en ce que l'effet thermique est obtenu par un laser dont l'irradiance est supérieure ou égale à 0,5 W/cm², avantageusement supérieure ou égale à 10 W/cm² et encore plus avantageusement supérieure ou égale à 100 W/cm².

20. Procédé selon l'une quelconque des revendications 1, 16 à 19, caractérisé en ce que les chromophores sont choisis parmi des chromophores minéraux, tels que le noir de carbone, le graphite, l'oxyde de fer noir et l'oxyde de fer rouge, et des chromophores organiques, tels que la mélanine, le vert d'indocyanine, les colorants ou tout autre entité chimique inerte absorbant de façon suffisante à la longueur d'onde considérée.

21. Procédé selon la revendication précédente, caractérisé en ce que les chromophores sont des chromophores minéraux.

22. Procédé selon l'une quelconque des revendications précédentes 1, 16 à 21, caractérisé en ce que les chromophores et/ou la composition les comprenant sont choisis et/ou formulée de telle sorte qu'il n'y ait pas pénétration des chromophores à travers la peau.

23. Procédé selon l'une quelconque des revendications précédentes 1, 16 à 22, caractérisé en ce que, avant application du rayonnement laser, on applique une composition, plus particulièrement une composition filmogène, n'absorbant pas la lumière à la longueur d'onde utilisée par dessus la composition comprenant au moins un chromophore.

24. Procédé selon l'une quelconque des revendications précédentes 1, 16 à 23, caractérisé en ce qu'on utilise des lasers émettant dans le spectre visible, tels que, plus particulièrement, le laser à colorant pulsé (585 nm), le laser Rubis (694 nm) et le laser Nd:YAG doublé (532 nm).

25. Procédé selon l'une quelconque des revendications précédentes 1, 16 à 23, caractérisé en ce qu'on utilise des lasers émettant dans le spectre infrarouge, tels que, plus particulièrement, les lasers CO₂ (10,6 µm), Er:YAG (2,94 µm), Ho:YAG (2,12 µm) et Nd:YAG (1,06 µm).
